# EUROPEAN PATENT APPLICATION

(11) **EP 2 223 669 A1**
(43) Date of publication of application: **01.09.2010**
(21) Application number: 10153263.8
(22) Date of filing: 11.02.2010
(51) Int. Cl.: A61F 5/02, A61F 5/03

(54) **Highly adjustable lumbar support and methods**

(30) Priority: 27.02.2009 US 394867
(71) Applicant: CARSAR, LLC, Seal Beach, CA 90740 (US)
(72) Inventor: Garth, Geoffrey C., Long Beach, CA 90803 (US); Romo, Albert Victor, Lakewood, CA 90713 (US); Burke, Steven Richard, Huntington Beach, CA 92646 (US); Perez, Joel, Long Beach, CA 90814 (US)
(74) Representative: Rupp, Christian

(57) **Abstract**

A body brace for a wearer has a combination of adjustment mechanisms and fasteners to help fit the body brace to a wearer. A wearer first fastens the body brace about his abdomen and lumbar regions to generally fit the body brace around the wearer's waist. The wearer then adjusts one set of adjustment mechanisms that adjust an upper and lower circumference of the brace to allow the brace to fit both straight and pear-shaped bodies. Lastly, the wearer adjusts a second set of adjustment mechanisms that tighten a rear panel of the body brace against a lumbar region of the wearer. Preferably, the adjustment mechanism that is used to tighten the rear panel is an advantaged adjustment mechanism to allow for fine-tuning and a snug fit.

## Description

This application is a continuation-in-part of non-provisional application no. 10/977726 filed March 2, 2005, which is a divisional of non-provisional application no. 10/440525, filed May 19,2003.

### Field of the Invention

The field of the invention is lumbar supports.

### Background

Orthotic devices are typically provided for partial or substantial immobilization of the torso to stabilize the back. Some orthotic devices are back braces that fit around the torso around the lumbar area. When worn properly, a body brace can lend additional support to the abdomen and the spinal column to achieve spinal stability. However, for many users body braces are difficult to appropriately position and fasten. For example, US4640269 to Goins provides a back brace that is tightened around the body by threading a Velcro strap through a loop and pulling the strap backwards towards the user's posterior. The awkward angle of the strap prevents users from fully tightening the strap themselves and requires a third party to assist in the tightening process.

Goins and all other extrinsic materials identified herein are incorporated by reference in their entirety. Where a definition or use of a term in an incorporated reference is inconsistent or contrary to the definition of that term provided herein, the definition of that term provided herein applies and the definition of that term in the reference does not apply.

US 2007/0017945 to Willis teaches a body brace that wraps around a user's lumbar region and has a belt with a strap that pulls forward to fit the brace around the user. Willis' body brace is easier to tighten since the strap tightens by pulling forward, which is a natural body movement. Willis, however, fails to contour to body shapes of different shapes and sizes, for example differently shaped hips and different lordotic curves.

Thus, there is still a need in the art for a body brace that conforms to body shapes of different shapes and sizes.

### Summary of The Invention

The present invention provides apparatus and methods in which a body brace having a, multiple adjusting and a mechanically advantaged tightening mechanism.

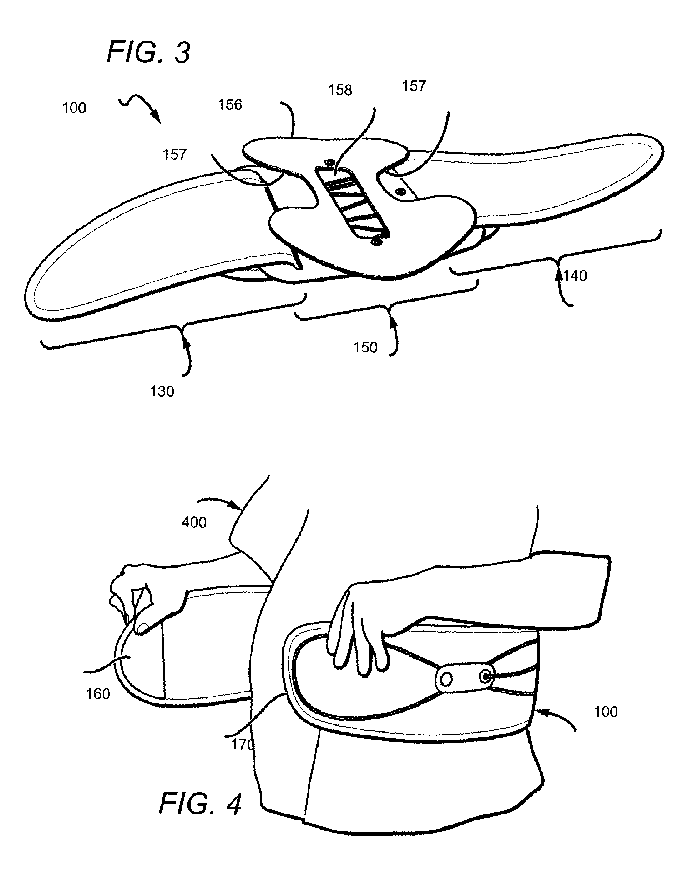

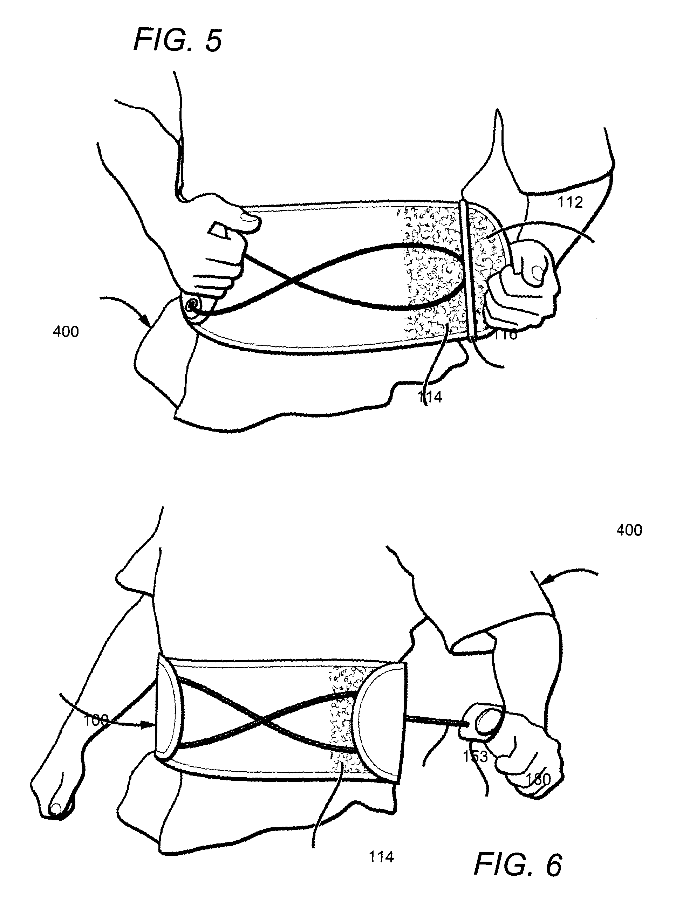

Braces can be applied to a wearer using three conceptually different actions, fitting, adjustment, and tightening, and preferred embodiments have separate mechanisms to accomplish those different actions. The initial fitting step involves coupling the two anterior ends of the side panels across the wearer's abdomen. In some cases this will involve a Velcro^{™} attachment of one end to the other, as shown in **Figure 4****.** In other cases this might involve coupling the two ends to an abdominal support plate or other component. The adjustment step involves folding an end of each of the side panels over itself as shown in **Figure 5**. Each end is preferably Velcro-ed directly onto a middle portion of the side panel, although it is possible to include an intermediate piece disposed between the end and the middle. The tightening step involves pulling on the pull tabs that operate the mechanically advantaged mechanism at the back of the brace, as shown in **Figure 6****.**

The section of the circumference with the fold-back mechanism is preferably at least 12 inches (30.48 cm) long, and could conceivably fold back along its entire length if need be, so as to accommodate a greater number of body sizes. In this instance, and where other upper limits are not expressly stated, the reader should infer a reasonable upper limit. In this instance, for example, a reasonable upper limit is about 80 inches (203.2 cm). Preferably, the fold-back adjustment mechanism are capable of adjusting the circumference of the body brace down from a useable maximum circumference by at least 30 or 40 percent, and in the case of a double-fold back mechanism by at 50 or even 60 percent.

Unless a contrary intent is apparent from the context, all ranges recited herein are inclusive of their endpoints, and open-ended ranges should be interpreted to include only commercially practical values.

In a preferred embodiment, the fold-back mechanisms use a Velcro^{™} or other hook and loop fastener to fasten an end of the fold-back mechanism back on itself. Other fasteners are also contemplated, however, including for example belt buckles, buttons, push-push connectors, and ratchet buckles. The fold-back mechanism is preferably at least 2 inches (5.08 cm) high, and can fasten at an angle to itself so that the upper circumference of the body brace can be altered relative to the lower circumference of the body brace. In an exemplary embodiment, the fold-back adjustment mechanism could be coupled to a pivot such that when the fold-back adjustment mechanisms rotates upward or downward, the ratio between the upper circumference of the body brace changes relative to the lower circumference. Preferably, the fold-back mechanism rotates about the pivot by at least 15, 30, 45, or even 60 degrees upward or downward. The angular fastening mechanism and/or pivoting of the fold-back mechanism allows the body brace to be adjusted to fit cylindrically shaped wearers as well as pear shaped wearers. One, two, three, four, or more fold-back adjustment mechanisms could be distributed about the circumference of the body brace to allow for fine-tuning of the length or angle of the circumference of the body brace.

With multiple adjustment mechanisms being used to adjust the upper and lower circumferences of the brace, a proper adjustment of an upper and lower circumference about a wearer may take some time to achieve. Preferably, once the circumference adjustment mechanisms are set, the wearer can then get in and out of the brace using the fitting mechanism shown in Figure 4, or other quick release buckle, button, or other fastener that fastens and unfastens the body brace about the wearer. Preferably, the quick-release is coupled to one of the fold-back adjustment mechanisms as a pivot, providing dual functionality.

An exemplary fold-back adjustment mechanism includes a slot in the front or back support piece and a long side piece that threads through the slot and folds back upon itself for fastening. As used herein, "fastening" means that the side piece could attach directly to a portion of itself, or to an intervening material that is coupled to a portion of itself. A fold-back mechanism could also attach to other materials or portions of the body brace, including other fold-back mechanisms.

A front support piece could be used that places a posterior force on the abdominal region of the wearer, and could be shaped and disposed to contact a large area of the wearer's abdominal region. In a currently contemplated embodiment, the contact area preferably has a height of at least 3 inches (7.62), 4 inches (10.16 cm), 6 inches (15.24 cm), or even 8 inches (20.32 cm). These specific dimensions are, however, considered design choices, and alternatively dimensioned front pieces are contemplated to adjust for different body shapes and sizes, pregnancy, and so forth. The contact surface area is preferably centered at the sagittal midplane of the wearer, centered about the wearer's belly button. Having a front support piece with a maximal contact surface area height of about 4-6 inches allows the front support piece to support a weak transverse abdominal muscle to provide additional back support.

A back support piece could also be used that places an anterior force on the lumbar region of the wearer, and could be shaped and disposed to not only contact a large area of the wearer's lumbar region, but provide lordosis support for a sacrum of the wearer. Preferably, an advantaged adjustment mechanism is used to adjust the shape and size of the back support piece. The advantaged adjustment mechanism could be a series of pulleys, gears, levers, screws, or combinations thereof to provide more force to the adjustment. Preferably, the advantaged adjustment mechanism is mechanically advantaged more than 2:1, and is more preferably mechanically advantaged at 4:1 or more. Additional pulleys or longer levers could easily increase the mechanical advantage ratio of the mechanism. The back piece could be rigid, or could have rigid components with joints that bend and flex depending on how the adjustment mechanism is manipulated. Preferably, multiple adjustment mechanisms adjust the shape and size of the back support. For example, an upper cord guide could conform the upper 3 inches (7.62 cm) of the back support to a lumbar-thoracic area, while a lower cord guide could conform the lower 3 inches (7.62 cm) of the back support to a lumbar-sacral area.

Preferably the adjustment mechanisms tighten when a wearer pulls a tightening element forward, so that tightening the adjustment mechanisms is easy for a wearer. For example, a side panel with a fold-back mechanism preferably folds forward, and a back panel with an advantaged pulley adjustment mechanism has strings that tighten when pulled forward. The tightening elements preferably have a fastener, for example Velcro^{™} hooks and loops, that mate with a side of the body brace.

Various objects, features, aspects and advantages of the present invention will become more apparent from the following detailed description of preferred embodiments of the invention, along with the accompanying drawings in which like numerals represent like components.

### Brief Description of the Drawing

Fig. 1 is a front perspective view of a body brace.

Fig. 2 is an exploded view of the back piece of the body brace of Figure 1.

Fig. 3 is a rear perspective view of the body brace of Figure 1.

Fig. 4 is a front perspective view of the body brace of Figure 1 being wrapped around a wearer.

Fig. 5 is a front perspective view of the body brace and wearer of Figure 4, with a fold-back adjustment mechanism being tightened.

Fig. 6 is a front perspective view of the body brace and wearer of Figure 4, with an advantaged adjustment mechanism being tightened.

Fig. 7 is a front perspective view of an alternative body brace.

Figs. 8A and 8B show a horizontal cross-section of the alternative body brace of Figure 7 with a double fold-back adjustment mechanism.

### Detailed Description

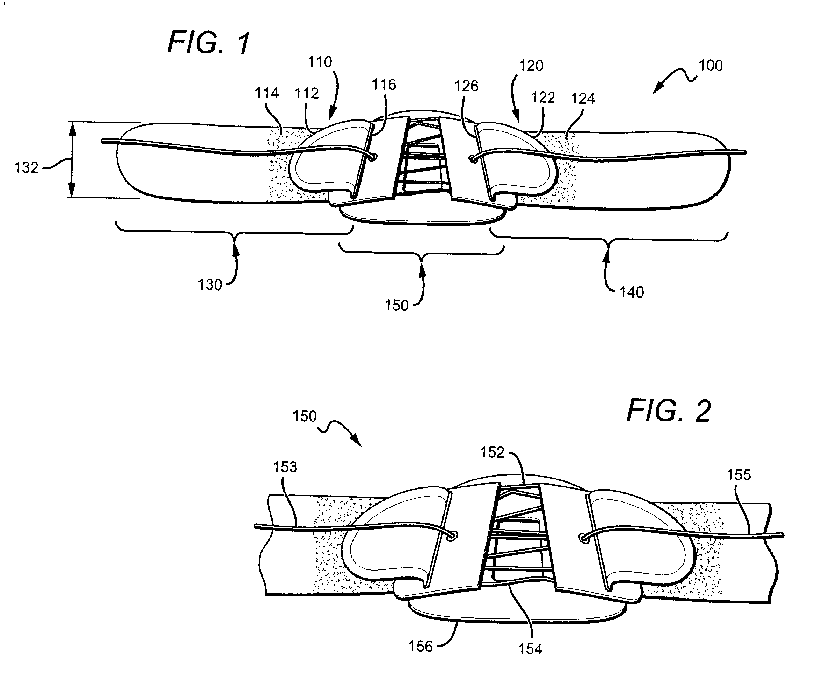

Referring to the drawings to illustrate preferred embodiments, but not for the purpose of limiting the invention, **Figure 1****,** illustrates one embodiment of a body brace 100 with a first adjustment mechanism 110, a second adjustment mechanism 120, a left side piece 130, a right side piece 140, and a back piece 150.

First adjustment mechanism 110 is a fold-back adjustment mechanism that adjusts an effective length of left side piece 130. As used herein, an "effective length" is the length of the side piece that acts as the circumference of the body brace when the brace is worn about a wearer. As best seen in Figure 1, a hook portion 112 of left side piece 130 threads through slot 116 of back piece 150. The hook portion 112 then folds back over a loop portion 114 of left side piece 130 which then mates with loop portion 114 to form a hook and loop fastener. Such hook and loop fasteners are commonly referred to in the art as Velcro^{™} fastening.

Preferably, the hook portion covers at most 4 in² or 9 in² of the left side piece while the loop portion covers most of the outside surface of the left side piece. By apportioning the hook portion and the loop portion in such a manner, the first adjustment mechanism could reduce the effective length of the first side piece by as much as 50%. While a hook and loop fastener is shown, other suitable fasteners are contemplated, for example belt buckles, buttons, push-push connectors, and ratchet buckles.

Slot 116 preferably has a height more than 10% or 20% greater than the height 132 of left side piece 130. A larger height allows the hook portion to be pulled up or down when it folds over itself, angling the left side piece with respect to the slot. When a wearer pulls hook portion 112 downwards before fastening, the effective length of the top of left portion 130 is smaller than the effective length of the bottom of left portion 130, which causes the upper circumference of the body brace to by smaller than the lower circumference of the body brace when the body brace is worn.

Second attachment mechanism 120 is similar to first attachment mechanism 110, and adjusts an effective length of right side piece 140. While second attachment mechanism 120 preferably operates in substantially the same manner as first attachment mechanism 110 to provide symmetry, second attachment mechanism 120 could differ in size, shape, and utility without departing from the scope of the present invention.

Left and right side pieces 130 and 140, respectively, are preferably made of synthetic polymer or a polymer netting to provide durability and flexibility, although other suitable materials are contemplated. Preferably, when the adjustment mechanisms are tightened, the material of left and right side pieces will remain taught against the sides of the wearer to provide support to the transverse abdominal muscles of the wearer. The loop portions of the left and right side pieces are preferably made of a soft material to prevent chaffing.

In **Figure 2**, an exploded view of back piece 150 has upper cord guide 152 and a lower cord guide 154 which adjusts an upper portion and a lower portion of panel 156. Upper cord guide 152 is an advantaged adjustment mechanism that mechanically advantages a force exerted on cord 153 by using cord guide lobes as pulleys. Cord 153 is threaded through three cord guide lobes (not shown) which multiply the linear motive force of a wearer by 4:1 when the wearer pulls on cord 153. While back piece 150 has two cord guides, each of which comprises three cord guide lobes, more or less cord guides and/or cord guide lobes could be used without departing from the scope of the present invention. As the cord guide lobes are pulled towards one another, panel 156 tightens and snugly hugs the lower back of the wearer. Preferably, cords 153 and 155 are positioned so that a user pulls the cords forward to tighten the advantaged adjustment mechanism against a back portion of the user. Exemplary cords have a handle with a fastener that mates with the sides of the body brace after tightening to hold the cords taught.

Cord guide systems and methods are explained in more detail in parent application serial no. 10/977726, which is incorporated herein by reference. Other suitable advantaged adjustment mechanisms are contemplated, for example gears, levers, screws, and combinations thereof. For example, a user could twist a crank located on the side panel that tightens the back panel via a gear mechanism.

The shape of back panel 156 is seen more clearly in **Figure 3****,** which has an upper and a lower flexible contour that tighten about a wearer's back when the cords are tightened. Recesses 157 and hole 158 create voids in back panel 156 which causes the center of back panel 156 to bend into a lordosis of the wearer. The back panel could be shaped in other suitable ways to provide back support to the wearer, including providing a combination of rigid and flexible sections.

In **Figures 4-6****,** a wearer 400 puts on body brace 100 and adapts the body brace according to his body type. First, in Figure 4, wearer 400 fits the body brace 100 by wrapping the brace around himself, and attaches first front fastener 160 to second front fastener 170, fitting the body brace about his waist area. This attachment system also provides a way to form the brace into either a cyllinder, a cone, or an inverted cone, depending on the angle made by the ends, alowing it to properly fit a range of patient physiologies. Front fasteners 160 and 170 are preferably hook and loops fasteners, although other contemplated fasteners could be used. In the absence of a separate front piece, front fasteners 160 and 170 cooperate to act as a front portion that provides a posterior force against the abdominal region of the wearer when fastened together. The front fasteners 160, 170 are preferably at least 4 inches (10.16 cm) or 6 inches (15.24 cm) tall about the wearer's belly button to provide a suitable posterior force. A separate, more rigid front panel (not shown) could be sewn or positioned into the body brace to provide additional support.

In Figure 5, the upper and lower circumferences of body brace 100 is adjusted by threading hook portion 112 through slot 116 and pulling hook portion 112 forward to fasten to loop portion 114. The upper circumference and lower circumference of the body brace is adjusted by pulling the hook portion up, down, or straight before fastening. After the upper and lower circumference of the body brace is set, a wearer does not need to adjust the body brace in the future.

In Figure 6, the wearer then pulls on handle 180 connected to cord 153, which then tightens the advantaged adjustment mechanism against the back of the wearer. Preferably, handle 180 has hook fasteners (not shown) that help couple handle 180 to loop portion 114, keeping cord 153 taught after tightening. Preferably, the wearer tightens both cord 153 and 155 simultaneously.

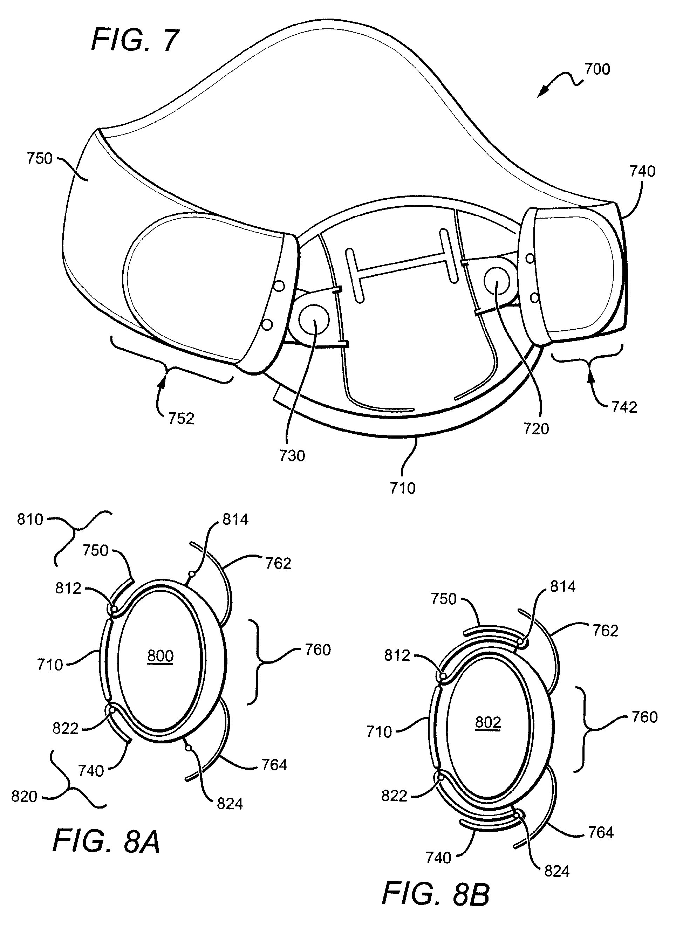

**Figure 7** shows an alternative body brace 700 with a an optional front piece 710 that places a posterior force on the abdominal region of the wearer. Like body brace 100 in Figure 1, left side piece 740 and right side piece 750 are adjusted using adjustment mechanisms 742 and 752, respectively. However, adjustment mechanisms 742 and 752 are coupled to front piece 710 with pivoting quick releases 720 and 730, respectively. By attaching the left and right side pieces to the front piece using pivoting quick-connects, the left and right side pieces could swing up or down along the pivot to change the upper and lower circumferences of the body brace relative to one another. Additionally, the quick-connect buckles could help a wearer quickly fit a body brace around his waist area and do a pivoting adjustment before tightening an advantaged adjustment mechanism (not shown).

Fold-back adjustment mechanisms that allow a part of the body brace to fold back over itself multiple times are also contemplated to fit a larger variety of wearers. **Figures 8A and 8B**, for example, show the alternative body brace 700 from Figure 7 with double fold-back adjustment mechanisms 810 and 820. Alternative body brace 700 has back support 760 and advantaged adjustment cords 762 and 764, and also has loops 812, 814, 822, and 824. As shown in Figure 8A, body brace 700 is fitted around large wearer 800 by threading left side piece through loop 822 and folding over itself. In Figure 8B, however, body brace 700 is fitted around small wearer 810 by threading left side piece through loop 822 and over itself, then through loop 824 and over itself. This allows the left and right side pieces to adjust to a wide variety of circumferences. In preferred embodiments the use of two double fold-back adjustment mechanisms are together capable of adjusting the circumference of the brace downward by at least 50%, and even more preferably at least 60%.

It should be apparent to those skilled in the art that many more modifications besides those already described are possible without departing from the inventive concepts herein. The inventive subject matter, therefore, is not to be restricted except in the spirit of the appended claims. Moreover, in interpreting both the specification and the claims, all terms should be interpreted in the broadest possible manner consistent with the context. In particular, the terms "comprises" and "comprising" should be interpreted as referring to elements, components, or steps in a non-exclusive manner, indicating that the referenced elements, components, or steps may be present, or utilized, or combined with other elements, components, or steps that are not expressly referenced. Where the specification claims refers to at least one of something selected from the group consisting of A, B, C .... and N, the text should be interpreted as requiring only one element from the group, not A plus N, or B plus N, etc.

## Claims

1. An abdominal compression brace configured to provide support to a wearer, comprising:
a first fold-back adjustment mechanism;
a second fold-back adjustment mechanism;
a tightening mechanism that is mechanically advantaged more than 2:1 and
a front portion that places a posterior force on an abdominal region of the wearer.

2. The abdominal compression brace of claim 1, wherein the first and second fold-back adjustment mechanism together are capable of adjusting a circumference of the brace downward by at least 30 percent, preferably by at least 50 percent.

3. The abdominal compression brace of claim 1, wherein the first fold-back adjustment mechanism comprises a slot in the front piece and a side piece that threads through the slot and folds back upon itself.

4. The abdominal compression brace of claim 3, wherein the first fold-back adjustment mechanism comprises a second slot in the side piece to form a double fold-back adjustment mechanism.

5. The abdominal compression brace of claim 3, wherein the second fold-back adjustment mechanism comprises a second slot in the front piece and a second side piece that threads through the second slot and folds back upon itself.

6. The abdominal compression brace of claim 1, wherein the front piece has a contact area height of at least 4 inches (10.16 cm), preferably at least 6 inches (15.24 cm).

7. The abdominal compression brace of claim 1, wherein a portion of the first fold-back adjustment mechanism folds over a portion of the second fold-back adjustment mechanism.

8. The abdominal compression brace of claim 1, wherein the first fold-back adjustment mechanism comprises a hook and loop fastener.

9. The abdominal compression brace of claim 1, further comprising a third fold-back adjustment mechanism.

10. The abdominal compression brace of claim 1, further comprising a fourth fold-back adjustment mechanism.

11. The abdominal compression brace of claim 1, further comprising a back piece that places an anterior force on a lumbar region.

12. The abdominal compression brace of claim 1, further comprising a rigid back piece that provides lordosis support for a sacrum of the wearer.

13. The abdominal compression brace of claim 1, wherein the tightening mechanism composes a back piece that conforms to a lordosis of the wearer.

14. The abdominal compression brace of claim 1, wherein the tightening mechanism comprises a cord guide, or upper and lower cord guides.

15. The abdominal compression brace of claim 1, further comprising a quick-release coupled to the first fold-back adjustment mechanism.

16. The abdominal compression brace of claim 1, further comprising a pivot coupled to the first fold-back adjustment mechanism.

17. The abdominal compression brace of claim 16, wherein the first fold-back adjustment mechanism rotates about the pivot by at least 30 degrees, preferably at least 45 degrees.
